(19) Europäisches Patentamt

European Patent Office

Office européen des brevets

(11) **EP 0 995 453 A1**

(12) **EUROPEAN PATENT APPLICATION**
published in accordance with Art. 158(3) EPC

(43) Date of publication:
**26.04.2000 Bulletin 2000/17**

(51) Int. Cl.$^7$: **A61M 5/14**, A61M 25/00,
A61M 19/00

(21) Application number: **98919517.7**

(22) Date of filing: **08.05.1998**

(86) International application number:
**PCT/JP98/02038**

(87) International publication number:
**WO 99/58176 (18.11.1999 Gazette 1999/46)**

(84) Designated Contracting States:
**DE FR GB IT**

(71) Applicant: **Dr. Japan Co. Ltd.**
**Tokyo 160-0022 (JP)**

(72) Inventors:
• **HIGUCHI, Akio**
**Dr. Japan Co. Ltd.**
**Tokyo 160-0022 (JP)**

• **HYUGAJI, Hayato**
**Dr. Japan Co. Ltd.**
**Tokyo 160-0022 (JP)**

(74) Representative:
**Altenburg, Udo, Dipl.-Phys.**
**Patent- und Rechtsanwälte**
**Bardehle . Pagenberg . Dost . Altenburg .**
**Geissler . Isenbruck**
**Postfach 86 06 20**
**81633 München (DE)**

(54) **MEDICAL PUNCTURE NEEDLE AND METHOD OF MANUFACTURING SAME**

(57)     A medical, bevel-shaped puncture needle (1) is provided such that squeezing work is applied to the fore-end part of a hollow round columnar part (2) of a needle blank (10) to form a hollow conical part (3) that is tapered off from the hollow round columnar part towards the tip thereof, and cutting work is then applied to the needle blank (10) to form a cutting face (4) crossing the needle axis (C) diagonally, with the fore end (5) thereof being located on the outer circumference of the hollow conical part and the rear end (6) thereof being located on the outer circumference of the hollow round columnar part.

The tip angle of the puncture needle (1) in a plane containing the tip thereof and the needle axis, is the sum of an inclination angle θ, relative to the needle axis (C), of the cutting face (4), and an inclination angle φ, relative to the needle axis (C), of a generatrix of the hollow conical part (3), that inclines towards the opposite side of the cutting face (4); and the direction of the bisector of the tip angle in which the puncture needle (1) proceeds when penetrating, coincides with, or is in close proximity to, the direction of needle axis.

# Fig.1

EP 0 995 453 A1

**Description**

Technical Field

The present invention relates to an improvement to a bevel-shaped puncture needle used for applying anesthesia and the like.

Background Technology

When pushed into, or penetrating, an affected part, a puncture needle proceeds substantially in the direction of the bisector of the tip angle of the puncture needle in a plane containing the tip of the puncture needle and the needle axis.

A bevel-shaped puncture needle has a configuration formed by diagonally cutting a needle tube of round columnar shape so that a cutting face is inclined at an angle of 15 degrees or more relative to the needle axis, and then by applying grinding work to both sides of the fore end of the cutting face to provide the fore end with an open angle of about 75 degrees. Because the tip angle of a puncture needle is defined by a generatrix of the round columnar surface parallel to the needle axis, and the major axis of the cutting face inclined relative to the needle axis, the tip angle is equal to the inclination angle of the cutting face relative to the needle axis, whereby the direction of the bisector of the tip angle makes an angular deviation of 7.5 to 10 degrees from the direction of the needle axis, towards the opposite side of the cutting face. Thus, when penetrating an affected part, the bevel-shaped puncture needle proceeds in a direction inclined at an angle of 7.5 degrees or more with the direction of the needle axis, towards the opposite side of the cutting face. Because of this, a problem existed that a bevel-shaped puncture needle easily misses the puncture target when the target is located in the depth.

To solve this problem, certain puncture needles are being used where the inclination angle of the cutting face of the bevel-shaped puncture needle is made a sharp angle of about 12 degrees to lessen the angular deviation of the proceeding direction of the puncture needle from the direction of the needle axis. Such puncture needles, however, have caused a problem of damaging tissues because the puncture needle easily penetrates more into the depth than needed due to the tip angle being too sharp.

Disclosure of The Invention

An object of the present invention is to have the proceeding direction, when penetrating, of a bevel-shaped puncture needle substantially coincided with the direction of the needle axis without making the tip of the puncture needle sharp-angled.

For this, a bevel-shaped puncture needle of the present invention has the fore-end part of the outer circumferential surface on the opposite side of the cutting face, formed into a conical surface by squeezing a needle tube of round columnar shape. Thereby, the tip angle of the puncture needle is defined, in a plane containing the tip and the needle axis, by the major axis of the cutting face inclined relative to the needle axis, and a generatrix of the conical surface that inclines towards the opposite side of the cutting face. Because this tip angle is the sum of the inclination angle, relative to the needle axis, of the cutting face, and the inclination angle, relative to the needle axis, of the generatrix of the conical surface, the tip angle is greater than that of conventional bevel-shaped puncture needles by the inclination angle of the conical surface when the inclination angle of the cutting face is made equal to that of the conventional bevel-shaped puncture needles. Therefore, it is not likely that the penetrating depth is greater than needed due to the tip angle being too sharp. The inclination angle of the conical surface of the fore-end part with the needle axis, however, is preferred to be made not greater than the inclination angle of the cutting face, because if the tip angle is too obtuse, the penetrating resistance gets higher.

When penetrating an affected part, the proceeding direction of a puncture needle of the present invention is, or is close to, the direction of the bisector of the tip angle. The inclination angle of this bisector inclining towards the opposite side of the cutting face, relative to the direction of the needle axis, is half of the difference between the inclination angles of the cutting face and the conical surface. Thus, the angular deviation of the proceeding direction of the puncture needle of the present invention from the direction of the needle axis, is by far smaller compared with the conventional bevel-shaped puncture needles, where the inclination angle of the bisector of the tip angle was half of the inclination angles of the cutting face. Therefore, the bevel-shaped puncture needle of the present invention is most unlikely to miss the puncture target.

In the case that the inclination angle of the conical surface of the fore-end section of a puncture needle of the present invention is set equal to the inclination angle of cutting face, the direction of the bisector of the tip angle coincides with the direction of the needle axis. Therefore, if the puncture needle vertically penetrates skin, directly under which in the depth a puncture target is located, the puncture needle proceeds straight in the direction of the needle axis to ensure to penetrate the puncture target in the depth.

A puncture needle of the present invention is manufactured from a needle blank of metallic round tube. In the first process, by applying squeezing work to the fore-end section of the needle blank, a truncated conical fore-end part is formed that has a specified inclination angle, e.g., equal to or smaller than the inclination angle of a cutting face, relative to the needle axis. In the second process, the needle blank is cut along a plane that extends through the truncated conical fore-end part and crosses the needle axis at the inclination angle of the cutting face, to form the cutting face, with the rear end thereof crossing the round columnar part of the needle blank, and the fore end thereof crossing the conical part of the fore end of the needle tube.

The cutting face of the bevel-shaped puncture needle manufactured in this way, is of a configuration of ellipse with one side in the direction of major axis thereof rounded, where the opposite side of the cutting face is a part of the circumferential surface of the round column except the fore end and vicinity thereof, while the fore end and vicinity thereof is a part of the circumferential surface of the conical part. Furthermore, the distance from the needle axis to the rear end of the cutting face is equal to the radius of the needle blank while the distance therefrom to the fore end of the cutting face is smaller than the radius of the needle blank. The tip angle of this puncture needle is the sum of the inclination angle of the cutting face relative to the needle axis and the inclination angle of the conical part of the fore end of the needle.

Brief Description of The Drawings

FIG. 1 is a longitudinal sectional view showing, enlarged, a principal part of a puncture needle of an embodiment of the present invention,
FIG. 2 is a plan view of the puncture needle of FIG. 1,
FIG. 3 is a view corresponding to FIG. 1 of another embodiment, and
FIG. 4 is illustrations showing a manufacturing process of the embodiment of FIG. 1.

Best Embodiments of The Invention

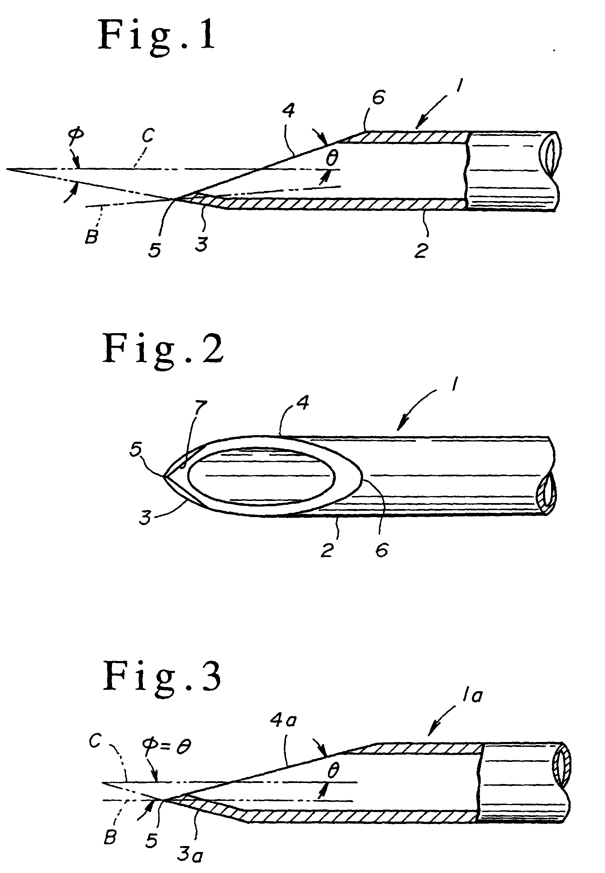

As shown in FIGs. 1 and 2, a puncture needle 1 is made of stainless steel tube, which is a hollow round columnar part 2 except the fore-end section. The fore-end section of the puncture needle 1 is a hollow conical part 3 formed by squeezing the hollow round columnar part 2 so as to successively taper off towards the fore-end side. Furthermore, chamfers 7 are provided to both sides of the tip by means of grinding so that the tip of a cutting face has a specified open angle.

The cutting face 4 crosses the needle axis C at an inclination angle $\theta$, intersecting the hollow round columnar part 2 and the hollow conical part 3 diagonally. A generatrix of the hollow conical part 3 crosses the needle axis C at an inclination angle $\phi$. Because the fore end 5 of the cutting face is located on the outer circumferential surface of the hollow conical part, and the rear end 6 thereof located on the outer circumferential surface of the round columnar part 2, the distance from the fore end 5 to the needle axis is smaller than the radius of the hollow round columnar part 2.

The tip angle of the cutting face 4 in a plane containing the fore end 5 and the needle axis C, is the sum of the inclination angle $\theta$ of the cutting face 4, and the inclination angle $\phi$ of the generatrix of the hollow conical part 3 on the opposite side of the cutting face, i.e., $\theta+\phi$.

If the inclination angle $\theta$ of the cutting face 4 is set equal to or smaller than a conventional inclination angle of the cutting face, and the inclination angle $\phi$ of the hollow conical part 3 is set smaller than $\theta$, the tip angle of the puncture needle 1 is greater than, but not greater than twice, the conventional inclination angle of the cutting face. Thus, the puncture needle 1 of such tip angle is neither likely to penetrate an affected part too deep due to the tip angle being too sharp, nor likely to cause too much penetrating resistance due to the tip angle being too obtuse.

When this puncture needle 1 penetrates an affected part, the puncture needle 1 proceeds in the direction of, or close to, the direction of the bisector of the tip angle. The inclination angle of the direction of this bisector B, relative to the direction of the needle axis C, is $(\theta-\phi)/2$, and therefore is by far smaller than the conventional inclination angle of the direction of the bisector, which is $\theta/2$, whereby this puncture needle 1 proceeds substantially in the direction of the needle axis C. Thus, if the direction of the needle axis is aligned with the puncture target, the puncture needle 1 is most unlikely to miss the puncture target.

As shown in FIG. 3, if the inclination angle $\theta$ of the cutting face 4a, relative to the needle axis, of the puncture needle 1a is set equal to the inclination angle $\phi$, relative to the needle axis, of the conical part 3a of the fore end, the bisector B of the tip angle is parallel to the needle axis C in a plane containing the fore end 5 and the needle axis C. When penetrating an affected part, this puncture needle 1a proceeds in the direction of the needle axis, and therefore easily penetrates a target in the depth under skin.

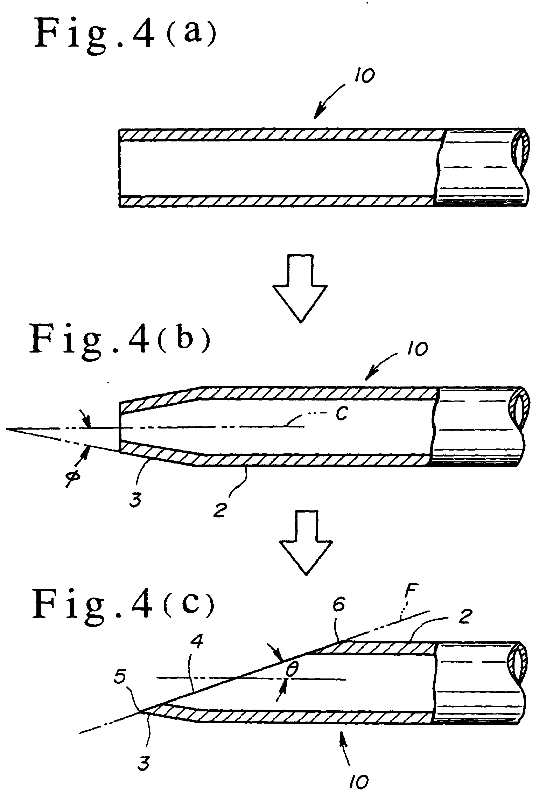

As shown in FIG. 4(a), a puncture needle of the present invention is manufactured from a needle blank 10 of hollow round column made of stainless steel tube.

In the first process shown in FIG. 4(b), by applying squeezing work to the fore-end section of the hollow round columnar part 2 of the needle blank 10, a hollow conical part 3 of hollow truncated conical shape is formed, a generatrix of which crosses the needle axis C at an inclination angle $\phi$.

In the second process shown in FIG. 4(c), the needle blank 10 is cut along a plane F that extends through both the hollow conical part and the hollow round columnar part 2, and crosses the needle axis C at an inclination angle $\theta$ of a cutting face 4, to form the cutting face 4, with the fore end 5 thereof located on the outer circumferential surface of the conical part 3, and the rear end 6 thereof located on the outer circumferential surface of the round columnar part 2. After that, work such as grinding, is applied for providing chamfers to the fore end, to finish a puncture needle 1 shown in FIG. 1.

In a puncture needle 1 manufactured in this way, the outer circumferential surface on the opposite side of the cutting face 4 is a part of the circumferential surface of the round columnar part 2 except the fore end and vicinity thereof, while the fore end and vicinity thereof is a part of the outer circumferential surface of the conical part 3. The tip angle of the cutting face 4 in a plane containing the fore end 5 and the needle axis C, is the sum of the inclination angle $\theta$ of the cutting face 4, and the inclination angle $\phi$ of the generatrix of the hollow conical part 3 inclining on the opposite side of the cutting face 4, i.e., $\theta+\phi$.

**Claims**

1. A medical, bevel-shaped puncture needle (1), having a cutting face (4) crossing a needle axis (C) diagonally, said puncture needle characterized in that:

   the opposite side of said cutting face (4) is a hollow round columnar part (2) except the fore-end part thereof, said needle axis (C) being the center of said hollow round columnar part;
   said fore-end part is a hollow conical part (3) formed by squeezing off said hollow round columnar part towards the fore end thereof;
   said cutting face (4) has the fore end (5) thereof located on the outer circumference of said hollow conical part, and the rear end (6) thereof located on the outer circumference of said hollow round columnar part; and
   the tip angle of said puncture needle in a plane containing said fore end (5) and the needle axis (C), is the sum of the inclination angle $\theta$, relative to said needle axis, of said cutting face, and the inclination angle $\phi$, relative to said needle axis, of a generatrix of said hollow conical part, that inclines towards the opposite side of said cutting face.

2. A medical, bevel-shaped puncture needle according to claim 1, characterized in that said inclination angle $\phi$ of said generatrix of said hollow conical part (3) made by crossing said needle axis (C), is equal to, or smaller than, said inclination angle $\theta$ of said cutting face made by crossing said needle axis (C).

3. A method of manufacturing a medical, bevel-shaped puncture needle (1), characterized in comprising the steps of:

   forming a hollow round columnar part (2) and a hollow conical part (3) that extends continuously from said hollow round columnar part towards the tip thereof, by applying squeezing work to the fore-end section of a needle blank (10) of metallic round tube; and

applying, then, cutting work to said needle blank (10) so as to form a cutting face (4) crossing the needle axis (C) at an inclination angle $\theta$, said cutting face having the fore end (5) thereof located on the outer circumferential surface of said hollow conical part, and the rear end (6) thereof located on the outer circumferential surface of said hollow round columnar part.

4. A method of manufacturing a medical, bevel-shaped puncture needle (1) according to claim 3, characterized in that said inclination angle $\phi$ of said generatrix of said hollow conical part (3) made by crossing said needle axis (C), is equal to, or smaller than, said inclination angle $\theta$ of said cutting face made by crossing said needle axis (C).

# Fig.1

# Fig.2

# Fig.3

# Fig.4 (a)

# Fig.4 (b)

# Fig.4 (c)

## INTERNATIONAL SEARCH REPORT

| | International application No. |
|---|---|
| | PCT/JP98/02038 |

| A. CLASSIFICATION OF SUBJECT MATTER |
|---|
| Int.Cl⁶ A61M5/14, 25/00, 19/00 |

According to International Patent Classification (IPC) or to both national classification and IPC

| B. FIELDS SEARCHED |
|---|
| Minimum documentation searched (classification system followed by classification symbols) |
| Int.Cl⁶ A61M5/14, 25/00, 19/00 |

Documentation searched other than minimum documentation to the extent that such documents are included in the fields searched

| Jitsuyo Shinan Koho | 1940-1998 | Toroku Jitsuyo Shinan Koho | 1994-1998 |
|---|---|---|---|
| Kokai Jitsuyo Shinan Koho | 1971-1998 | | |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| X | JP, 9-276403, A (Kawasumi Laboratories, Inc.), October 28, 1997 (28. 10. 97), Full text ; Particularly Figs. 1 to 3 ; Par. No. [0005] (Family: none) | 1-4 |
| X | JP, 33-14884, Y1 (Hachiko Denki Seisakusho K.K.), September 12, 1958 (12. 09. 58) (Family: none) | 1-4 |
| X | JP, 9-56815, A (Yoshikuni Saito), March 4, 1997 (04. 03. 97), | 1-2 |
| A | Claims ; Fig. 1 & AU, 9651910, A & US, 5575780, A & CA, 2174459, A | 3-4 |
| X | Microfilm of the specification and drawings annexed to the request of Japanese Utility Model Application | 1-2 |
| A | No. 81177/1974 (Laid-open No. 10896/1976) (Ryohei Kusanagi), January 27, 1976 (27. 01. 76), Particularly Fig. 1 & JP, 51-10896, U | 3-4 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| August 4, 1998 (04. 08. 98) | August 18, 1998 (18. 08. 98) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

International application No.

PCT/JP98/02038

| C (Continuation). DOCUMENTS CONSIDERED TO BE RELEVANT | | |
|---|---|---|
| Category* | Citation of document, with indication, where appropriate, of the relevant passages | Relevant to claim No. |
| X | JP, 45-31383, B1 (Minoru Tajima), October 12, 1970 (12. 10. 70) (Family: none) | 1-2 |
| A | | 3-4 |
| A | JP, 50-61092, A (Crinospital S.p.A.), May 26, 1975 (26. 05. 75) & IL, 45549, A & BE, 819324, A1 & FI, 254874, A & SE, 7412177, A & NO, 743421, A & NL, 7412663, A & DE, 2443199, A1 & DK, 510174, A & BR, 740798, A & FR, 2257310, A1 & US, 3924617, A & IT, 998674, A & AU, 7335274, A | 1-4 |

Form PCT/ISA/210 (continuation of second sheet) (July 1992)